# EUROPEAN PATENT APPLICATION

(11) **EP 2 679 144 A1**
(43) Date of publication of application: **01.01.2014**
(21) Application number: 12425118.2
(22) Date of filing: 27.06.2012
(51) Int. Cl.: A61B 1/267

(54) **Blade of a laryngoscope, or similar devices, with a progressive spreading mechanism**

(71) Applicant: De Domenico, Andrea, 00176 Roma (IT)
(72) Inventor: The designation of the inventor has not yet been filed
(74) Representative: Zizzari, Massimo

(57) **Abstract**

Blade (200) of a laryngoscope, or similar devices, having a progressive spreading mechanism, said blade (200) characterized in that comprising:
- a lower part (201), preferably rectangular, which extends along the longitudinal direction and having a profile that is curved at the end (204), in turn comprising: a first dividing wall (208) positioned transversely to said lower part (201), which extends longitudinally up to a specific distance, and having a series of primary protruding teeth (212a, 212b, 212c, etc.) positioned along one or more columns, and that regulate the transverse thickness;
- an upper part (202), preferably rectangular, which extends along the longitudinal direction and having a profile that is curved at the end (203), in turn comprising: a second dividing wall (209) positioned transversely to said upper part (202), which extends longitudinally up to a specific distance, and having a series of secondary protruding teeth (218a, 218b, 218c, etc.) positioned along one or more columns, that regulate the transverse thickness, and interlocking to said primary protruding teeth (212a, 212b, 212c, etc.);
- a series of cylindrical elements (205a, 205b, 205c, etc.) that are all connected each other in correspondence of their axis, using a flexible articulated structure (206) free to move, under control of an operator, along the longitudinal direction, i.e. the main axis of the blade (200), inside an operative channel defined by a closed space between the above-mentioned lower part (201) and the upper part (202) of the blade (200),
so that the sliding and rolling motion of said cylindrical elements (205a, 205b, 205c, etc.), inside the above-mentioned operative channel, cause the spreading by contact of the space between said end (204) of the lower part (201) in respect to said end (203) of the upper part (202) of the blade (200), with a consequent increase of thickness of the distal part of the same blade (200).

## Description

The present invention concerns a medical device, preferably applied to an *Airtraq®* tunneled *videolaryngoscope,* or a *LMA*® tunneled *videolaryngoscope,* or a Pentax AWS® *videolaryngoscope,* or other similar instruments, particularly suitable as a support to the procedure of a patient's tracheal intubation. In particular, the device includes two moving parts, an upper one and a lower one, whose relative motion along the transverse axis to the longitudinal axis, achieves a significant reduction of laminar thickness during the patient's intubation procedure. More in particular, during the phase of insertion of an instrument in the patient's mouth (oral cavity), a small inter-foreteeth distance could result in a critical factor that makes the insertion of the ordinary devices very difficult or even impossible. Instead, the proposed solution permits a proper placement of the distal part of the instrument inside the laryngeal cavity even in the above said situations. A following increase of thickness, resulting from the passage of cylindrical elements when the distal part of the instrument is correctly positioned in front of the glottic rhyme, permits to obtain a safer intubation procedure, even in difficult situations, because of a wider visual field and a lower external surface in contact with the patient, just at the time when the instrument should find a way inside narrow spaces.

The direct laryngoscopy is a technique that permits to visualize the laryngeal structures in a clear and straight way, and it is achieved by an instrument called *laryngoscope.* Since half a century, this technique made it possible to address the anesthetists operating around the world, in order to define a standard procedure of tracheal intubation addressed to place a tube in trachea, by which a patient can receive oxygen and at the same time can maintain protected air ways during the anesthesia.

Despite the improvement of such a technique, obtained at a global level by years of clinical practice, and despite the different shapes of laryngoscopic blades that have been designed and are available on the market, in same cases the direct laryngoscopy is not possible, because of particular anatomical parts in some persons, or because of particular situations that can happen in an emergency room.

In example, direct laryngoscopy is not possible when the alignment of the three patient's axis (oral axis pharyngeal axis and laryngeal axis) cannot be achieved by the standard actions that a physician knows and can do. Therefore, since a long time there was a widely recognized need to find some alternative techniques, so that an optimal visualization of the laryngeal structures can be achieved, and so that to use a laryngoscope minimizing the risk of damage of the patient's tissues.

When new technologies become available, with new advanced materials, the laryngeal structures had more chances to be properly visualized, even in a not direct way, by the so called indirect laryngoscopy. Therefore, the techniques of indirect laryngoscopy made it possible to visualize the laryngeal structures clearly, using a camera, or an optical fiber, or further a system of prisms with the distal elements that can be placed close to the tracheal cavity.

Among the devices that, at the present time, permit to achieve the indirect laryngoscopy technique, there are *Airtraq, Pentax AWS,* and *A.P. Advance LMA,* that contain the endotracheal tube in a tunnel that is integrated into the blade, so that a guidance can be achieved all along the path, until the tube exits with the far end directly in the proper position in trachea. Although this solution prevents possible risks arising from use of stylets, and therefore represents a more comfortable procedure of endotracheal intubation, according to an indirect laryngoscopy technique, however it is characterized by significant drawbacks.

First of all, the operator is forced to express strong actions of pulling and rotating the device, just as an attempt, sometimes not effective, to align properly the far end of the instrument to the tracheal axis. Such movements can lead to a series of complications for the patient due to the strong forces that are expressed on tissues in case the above alignment is not easy to be obtained. The most important complication is represented by the possible damage to the laryngeal structures. Other possible complications due to the excessive thickness of the instrument and to quick movements in cases of emergency are oedema, bleeding or damages to teeth or dentures, that may also be inhaled and produce serious problems of respiratory obstructions.

Furthermore, the requirement to insert the endotracheal tube in an operative channel, in order to follow a proper routing path, leads as a consequence to the definition of an increased instrument's thickness, and to an increased minimum inter-foreteeth distance, in the attempts to insert the same instrument into the patient's mouth, and/or leads to the use of different instruments of different size, according to the diameter of the endotracheal tube to be inserted.

Another innovation in the laryngoscopy field has been introduced with the use of a magnetic guidance system according to the principles and teachings of the European patent application EP 11425045 (De Domenico) and a support device for endotracheal tube according to the principles and teachings of the European patent application EP 11425158 (De Domenico). In fact, these inventions, allow to overcome the above said drawbacks because they enable the variability of possible directions for routing the endotracheal tube at each stage of advancement within the patient's mouth, under the control of a guiding mechanism, with a consequent simplification of the procedure and a considerable reduction of the blade thickness in the longitudinal direction, so that to allow the reduction of the minimal inter-foreteeth distance required to introduce the laryngoscope blade through the mouth of the patient.

The aim of this invention is to further reduce the minimal inter-foreteeth distance required to introduce the laryngoscope blade through the mouth of the patient, without compromising the advantages relating to the above-mentioned inventions of the magnetic guidance system (patent application EP 11425045, De Domenico) and of the device for endotracheal tube (patent application EP 11425158, De Domenico).

In particular, during the phase of passing through the patient's mouth (oral cavity), this invention allows the reduction of the laminar thickness in the distal part of the blade, that represents a critical step during the operations of correct positioning of the same blade, in order to allow the consequent increase of thickness when the distal end of the laryngoscope blade is already positioned inside the pharynx, exactly at the point when the increasing thickness of the laryngoscope enables the optimal visualization of the vocal cords (increasing the visual field), and at the same time the passage of the endotracheal tube side by side to the walls of the laryngoscope. The device is also versatile because it enables the use of tubes having different diameters.

The object of the present invention is therefore constituted by a device which overcomes all the above mentioned drawbacks, being versatile and able to be applied to many types of endotracheal intubation devices that are currently on the market.

Therefore, it is specific subject of the present invention a blade of a laryngoscope, or similar devices, having a progressive spreading mechanism, said blade **characterized in that** comprising:
- a lower part, preferably rectangular, which extends along the longitudinal direction and having a profile that is curved at the end, in turn comprising: a first dividing wall positioned transversely to said lower part, which extends longitudinally up to a specific distance, and having a series of primary protruding teeth positioned along one or more columns, and that regulate the transverse thickness;
- an upper part, preferably rectangular, which extends along the longitudinal direction and having a profile that is curved at the end, in turn comprising: a second dividing wall positioned transversely to said upper part, which extends longitudinally up to a specific distance, and having a series of secondary protruding teeth positioned along one or more columns, that regulate the transverse thickness, and interlocking to said primary protruding teeth;
- a series of cylindrical elements that are all connected each other in correspondence of their axis, using a flexible articulated structure free to move, under control of an operator, along the longitudinal direction, i.e. the main axis of the blade, inside an operative channel defined by a closed space between the above-mentioned lower part and the upper part of the blade,
so that the sliding and rolling motion of said cylindrical elements, inside the above-mentioned operative channel, cause the spreading by contact of the space between said end of the lower part in respect to said end of the upper part of the blade, with a consequent increase of thickness of the distal part of the same blade.

The present invention will now be described for illustrative but not limitative purposes, according to its preferred embodiments, with particular reference to figures of the enclosed drawings, wherein:
figure 1 is a front perspective view of a laryngoscope blade with a magnetic guiding mechanism for an endotracheal tube, according to prior art (application EP 11425045, De Domenico), applied to an Airtraq videolaryngoscope;
figure 2 is a lateral view of a laryngoscope blade like that of figure 1, applied to an Airtraq videolaryngoscope inserted in a tracheal cavity of a patient, the same being represented by a lateral sectioned view;
figure 3 is a front perspective view of a laryngoscope blade with a progressive spreading mechanism for an endotracheal tube, according to the present invention, applied to an Airtraq videolaryngoscope;
figure 4 is a rear perspective view of a laryngoscope blade, like that of figure 3, with a progressive spreading mechanism;
figures 5-12 are a sequence of lateral sectioned views of the same laryngoscope blade with progressive spreading mechanism, in which the inner elements are located at eight respective positions, from a backward position of figure 5 (closed blade) up to a forward position of figure 12 (open blade);
figures 13-15 are a sequence of rear perspective views of the same laryngoscope blade with progressive spreading mechanism of figures 3-12, in which the inner elements are located at three respective positions, from a backward position of figure 13 (blade going to be open) up to a forward position of figure 15 (full open blade);
figure 16 is a front perspective view of a laryngoscope blade with a progressive spreading mechanism, applied to an Airtraq videolaryngoscope, in which the inner elements are located at a forward position (open blade);
figure 17 is a front perspective view of the same laryngoscope of figure 16, in which the inner elements are located at a forward position (open blade), and in which the same elements achieve a function of magnetic guidance for an endotracheal tube;
figures 18-19 are two perspective views, according to different angles, of a blade with progressive spreading mechanism according to a different embodiment of the present invention, in which the inner elements are located at a backward position (closed blade);
figures 20-21 are two perspective views, according to different angles, of a blade with progressive spreading mechanism according to the same different embodiment of the present invention of figures 18-19, in which the inner elements are located at a forward position (open blade).

It is here underlined that only few of the many conceivable embodiments of the present invention are described, which are just some specific non-limiting examples, having the possibility to describe many other embodiments based on the disclosed technical solutions of the present invention.

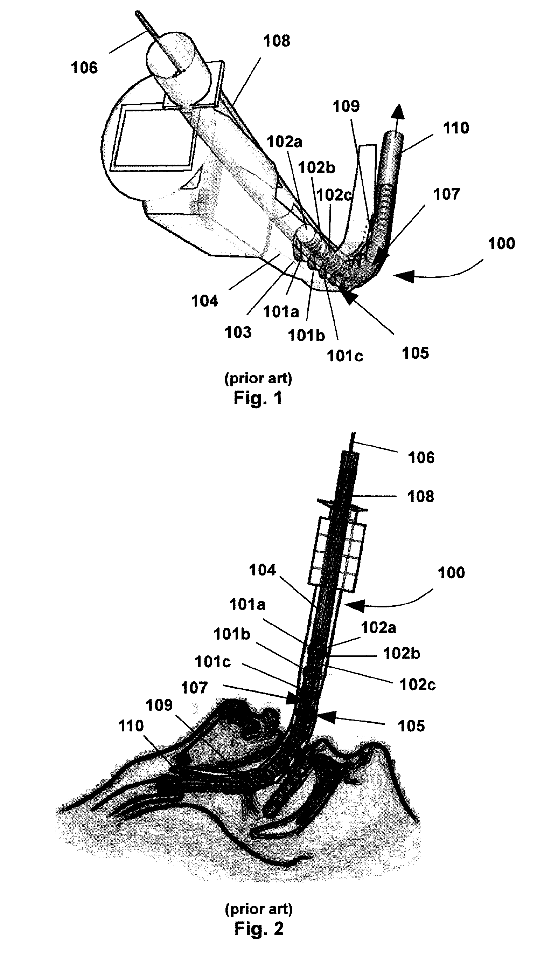

In figure 1 it is illustrated a device with magnetic elements 100, particularly suitable as a support to the procedure of tracheal intubation of a patient, embedded in the blade of a Airtraq® video-laryngoscope. The blade of the same laryngoscope, in the prior art has a longitudinal profile with some protruding walls, that achieves a guiding and routing tunnel, along which an endotracheal tube 108 is inserted until its final position, with the distal end in trachea, as illustrated in figure 2.

In the device 100 disclosed by patent application EP 11425045 (De Domenico), the protruding walls have been at least partially removed, and the guidance function is achieved by a *magnetic platform* 105, installed inside the blade, and interacting with a *magnetic train* 107 that has been previously inserted steadily inside the endotracheal tube 108.

The *magnetic platform* 105 comprises a first set of magnetic elements 101a, 101b, ..., etc., that are all connected each other, by a first flexible/articulated supporting structure 103; the *magnetic train* 107 comprises instead a second set of magnetic elements 102a, 102b, ..., etc., that are all connected each other, by a second flexible supporting structure 106. The elements 102a, 102b, ..., etc., of the *magnetic train* 107 have an opposite polarity in respect of elements 101 a, 101b, ..., etc., of said *magnetic platform* 105, so that a magnetic attractive force is generated by interaction all the time, keeping said endotracheal tube 108 strictly in contact with said *magnetic platform* 105. The *magnetic platform* 105 is free to move forward or backward, under control of an operator, along the longitudinal direction of an operative channel 104 belonging to the same device 100.

The basic principle of the present invention is to use the series of elements 101a, 101b, ..., etc., that can be made of magnetic material or not, in order to spread the distal part of the blade, so that when the elements 101a, 101b, ..., etc., are in a backward position the blade is closed and it takes a minimum transversal space, when the elements 101a, 101b, ..., etc., are in a forward position the blade is open and it takes a wide transversal space.

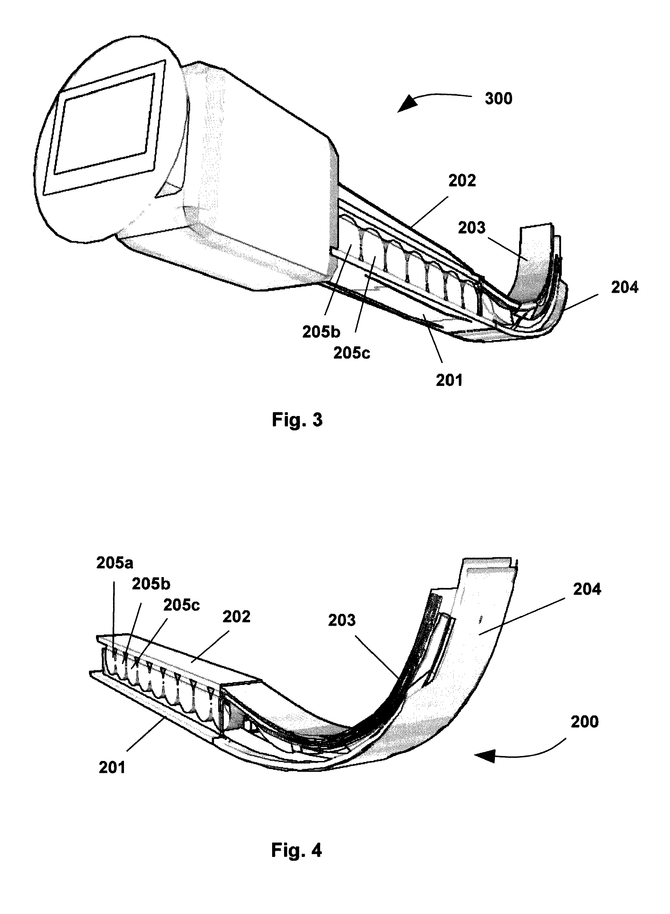

Figures 3-4 show a laryngoscope blade 200 with a progressive spreading mechanism for an endotracheal tube, according to the present invention, applied to an Airtraq videolaryngoscope 300.

The blade 200 essentially comprises: a lower part 201, preferably rectangular, which extends along the longitudinal direction and has a profile that is curved at the end 204; an upper part 202, either preferably rectangular, which extends along the longitudinal direction and has a profile that is curved at the end 203; and a series of cylindrical elements 205a, 205b, 205c, etc., that are all connected each other in correspondence of their axis, using a flexible articulated structure 206 free to move, under control of an operator, along the longitudinal direction, inside an operative channel. The operative channel is defined by a closed space between the above-mentioned lower part 201 and the upper part 202 of the blade 200.

The cylindrical elements 205a, 205b, 205c, etc., that are rigid in the longitudinal direction, and articulated in the transverse direction, are arranged transversely to the longitudinal direction of the above-mentioned operative channel in which they are inserted, so that they can roll / slide inside it, and in such a way to allow a reduced friction during their motion.

In this way, the sliding and rolling motion of said cylindrical elements 205a, 205b, 205c, etc., inside the above-mentioned operative channel, cause the spreading by contact of the space between said end 204 of the lower part 201 in respect to said end 203 of the upper part 202 of the blade 200, with a consequent increase of thickness of the distal part of the same blade 200.

The flexible articulated structure 206 provides connection of all the respective cylindrical elements 205a, 205b, 205c, etc., that are made of plastic or magnetic material, and it is free to move, under control of an operator, along the longitudinal direction, forwards or backwards, inside the operative channel belonging to the same blade 200, with a consequent spreading of the lower part 204 in respect to the upper part 203.

Furthermore, said flexible articulated structure 206 is entirely extractable / removable from the operative channel so that when the endotracheal tube 108 has been properly positioned in the tracheal cavity the removal of the blade 200 from the patient's mouth is easier, safe and simple.

As disclosed by patent application EP 11425045 (De Domenico), the same structure 206 could end, at the part that is closest to operator, with an upper surface that is coupled to gears of a toothed wheel; when the operator turns the toothed wheel backwards, the interconnected cylindrical elements 205a, 205b, 205c, etc., advance deeply in the blade 200, when the operator turns the toothed wheel forwards the interconnected cylindrical elements 205a, 205b, 205c, etc., move back towards the part that is closest to operator.

The toothed wheel further includes with a locking / safety mechanism which makes it impossible, when activated, the retraction and/or the advancement of said interconnected cylindrical elements 205a, 205b, 205c, etc.

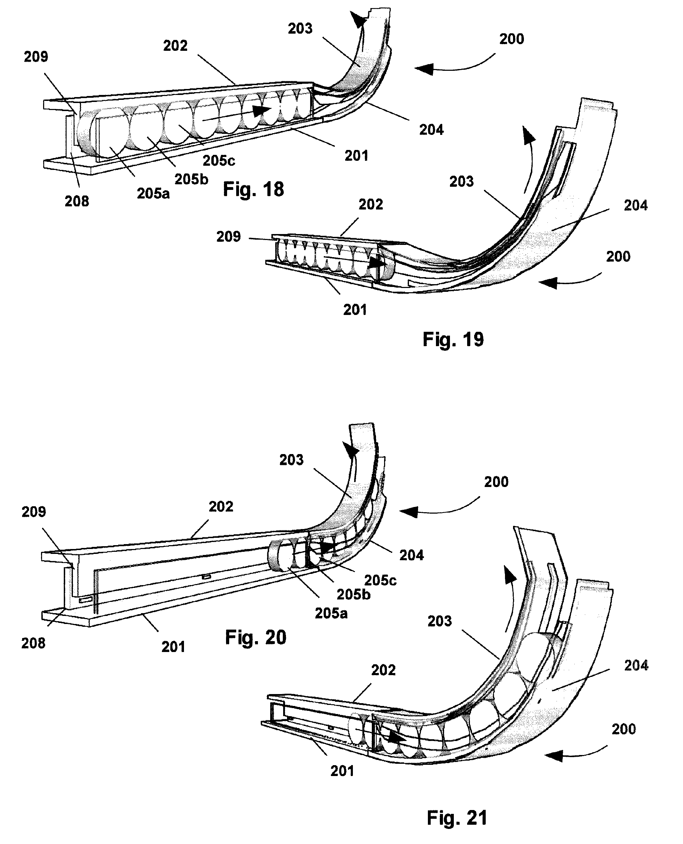

With reference to figures 18-21, the lower part 201 of blade 200 further comprises: a first dividing wall 208 positioned transversely to said lower part 201, which extends longitudinally up to a specific distance, and having a series of primary protruding teeth 212a, 212b, 212c, etc., positioned along one or more columns, and that regulate the transverse thickness; the upper part 202 further comprises: a second dividing wall 209 positioned transversely to said upper part 202, which extends longitudinally up to a specific distance, and having a series of secondary protruding teeth 218a, 218b, 218c, etc., positioned along one or more columns, that regulate the transverse thickness, and interlocking to said primary protruding teeth 212a, 212b, 212c, etc. In such a way, the device achieves a mechanism of adjustment of the size of blade, so that different types of endotracheal tubes, having different diameters, can be applied.

Figures 18 and 20 show a visual optical channel (if it is available, because optical devices could be contained, instead, inside the endotracheal tube), contained inside the first dividing wall 208 and suitable to transmit the images detected at the distal end of the blade 200, and the operative channel, in which the interconnected cylindrical elements 205a, 205b, 205c, etc., are free to move, that is perfectly / preferably parallel to the visual optical channel and that is placed in this case at the right side in respect of the point of view of operator. As an alternative, it could be placed on the left side, in order to facilitate in example the left-handed operators.

Figures 18-19 show the inner elements 205a, 205b, 205c, etc., located at a backward position, resulting in a closed blade with a distal part taking a minimum transversal space, instead figures 20-21 show the same inner elements 205a, 205b, 205c, etc., located at a forward position, resulting as a consequence in an open blade with a distal part taking a wide transversal space.

This transversal space can be further increased by embedding the so called *McCoy* function. A *McCoy laryngoscope* is known from the prior art as a specific device having a distal end of the blade that can be curved by moving mechanical means activated by a lever located on the handle of the same laryngoscope.

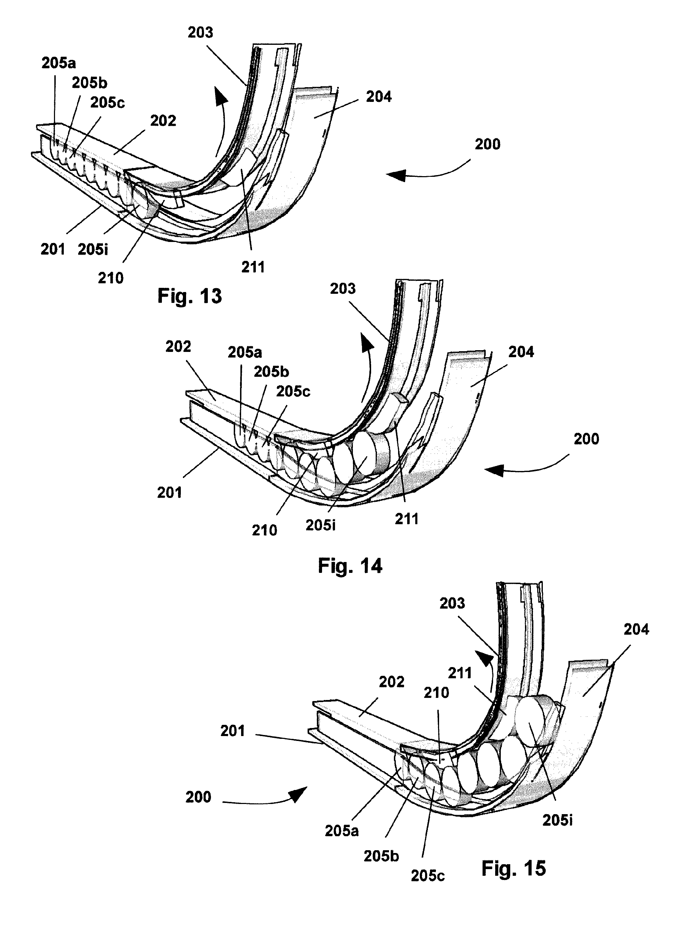

According to an alternative embodiments of the present invention, as represented in figures 13-15, one or more protruding elements 210, 211, are installed inside said operative channel so that, when they are intercepted by passage of said cylindrical elements 205a, 205b, 205c, etc., they achieve an additional spreading of the upper part 203 and the lower part 204 at the distal end of blade 200, embedding a mechanism of profile variation similar to that of a *McCoy laryngoscope.*

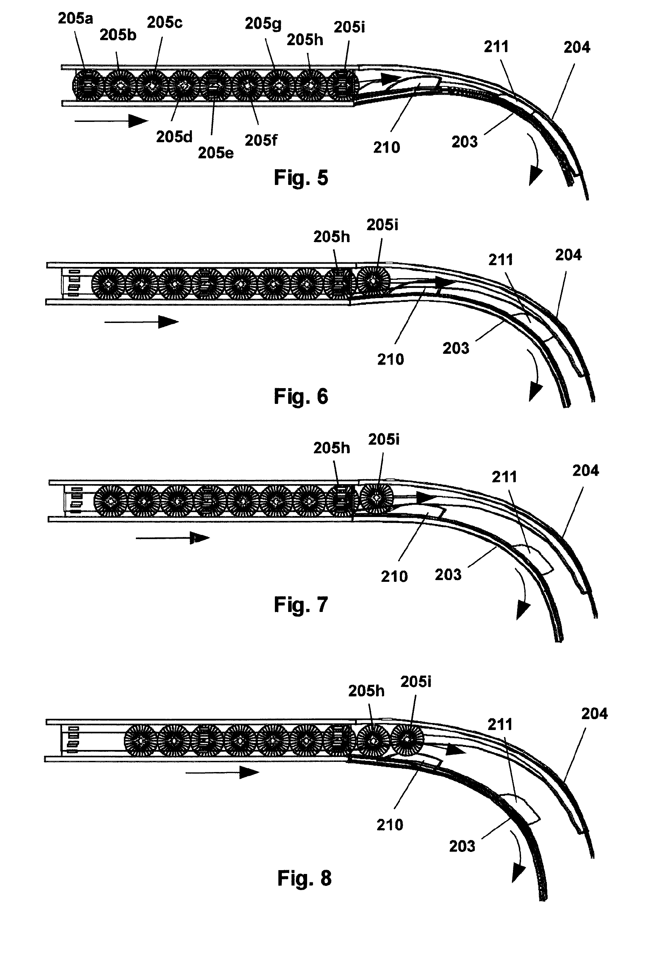

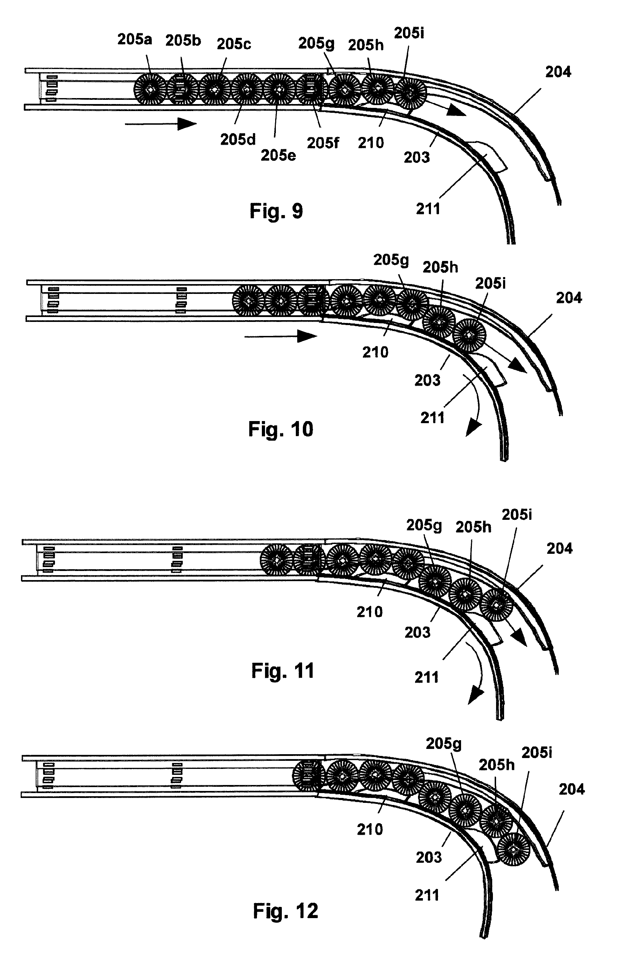

From the sequence shown in figures 5-12, it is better understood how, either in this case, a motion forward of elements 205a, 205b, 205c, etc., can cause the spreading by contact of the space between said end 204 of the lower part 201 in respect to said end 203 of the upper part 202 of the blade 200, with a consequent increase of thickness of the distal part of the same blade 200. At the point when the most advanced element 205i intercepts the first protruding element 210, it defines an additional spreading of the upper part 203 and the lower part 204, and when the same element 205i intercepts the second protruding element 211 (figures 11-12), the distal end goes to be further curved until it embeds a *McCoy* function.

One or more protruding elements (that could be parallel each other, jointed to the upper part and/or to the lower part of blade, but coupled each other so that they take as less space as possible when said cylinders are at backward position in the operative channel) can achieve a further spreading of the distal end of blade 200 that is close to the *pharynx,* without an additional increase of size of the other end of blade 200 that is instead close to the *oral cavity.*

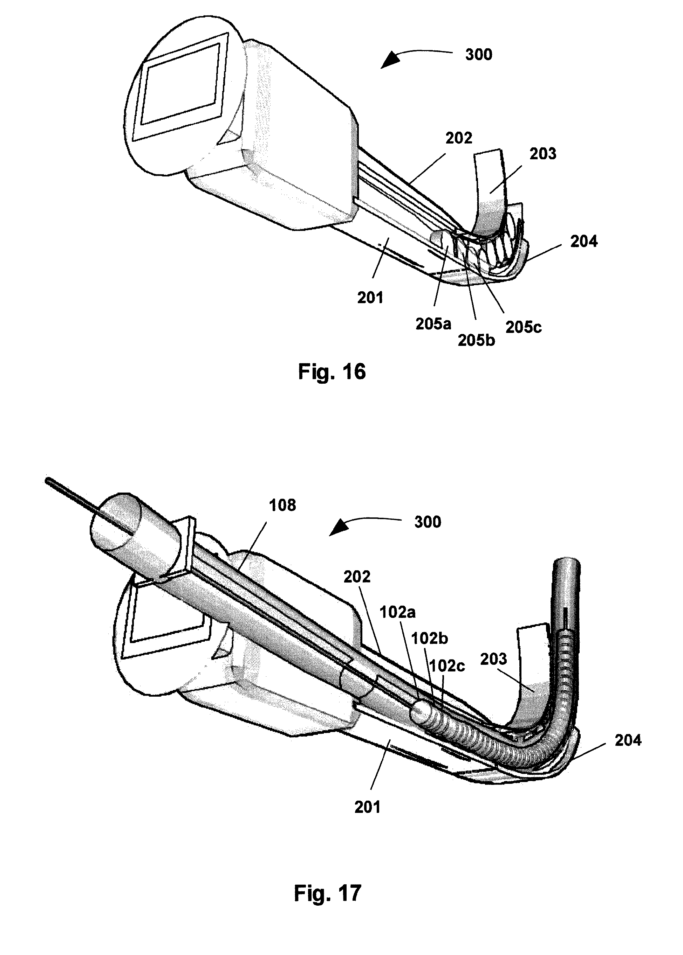

Figures 16-17 show how the above described mechanism of progressive spreading can be integrated with a guidance function for an endotracheal tube 108. In case the above said elements 205a, 205b, 205c, etc., are made of a magnetic material, they can interact with a series of further magnetic elements 102a, 102b, 102c, etc., generating an attractive magnetic force, that keeps the endotracheal tube 108 constantly in touch with the blade 200, embedding and improving the operative procedures disclosed by patent application EP 11425045 (De Domenico).

In particular, the rolling and sliding motion of the interconnected cylinders 205a, 205b, 205c, etc., cause the spreading by contact of the space between said end 204 of the lower part 201 in respect to said end 203 of the upper part 202 of the blade 200, with a consequent possibility of insertion of the endotracheal tube 108, immediately after the introduction of the same blade 200 inside the mouth of a patient.

Therefore, figure 3 shows a blade 200 in the first part of the intubation procedure, when the pharynx cavity has been not reached yet, instead figures 16-17 show the same blade in the last part of procedure, when the endotracheal tube 108 has been inserted with the distal end in the tracheal cavity.

According to another embodiment of the present invention, said endotracheal tube 108 can be housed in the middle, between operative channel and visual optical channel.

A disposable covering, preferably of plastic sterilized material, can cover said blade 200, so that to prevent possible infections and / or contamination; the same blade 200 can be disposable.

The above said lower part 201, upper part 202 as well as the blade 200, the first dividing wall 208 and the second dividing wall 209, can be composed of preformed metallic material that is easily adaptable to the specific anatomy of the patient.

The primary protruding teeth 212a, 212b, 212c, etc., and the secondary protruding teeth 218a, 218b, 218c, etc., are composed of the same metallic material by which said lower part 201 and said upper part 202 are made, and their operation is based on a coupling mechanism that adjusts the spreading between the two parts 201 and 202 on different levels of sizes, depending on the diameter of the endotracheal tube 108 to be applied.

Therefore, the above examples show that the present invention achieves all the proposed objectives. In particular, it discloses a blade that permits to overcome all the drawbacks of the prior art, achieving a great versatility of use, because it can be embedded into large part of the systems currently available on the market.

In particular, during the phase of passing through the patient's mouth (oral cavity), this invention allows the reduction of the laminar thickness in the distal part of the blade, that represents a critical step during the operations of correct positioning of the same blade, in order to allow the consequent increase of thickness when the distal end of the laryngoscope blade is already positioned inside the pharynx, exactly at the point when the increasing thickness of the laryngoscope enables the optimal visualization of the vocal cords (increasing the visual field), and at the same time the passage of the endotracheal tube side by side to the walls of the laryngoscope. The device is also versatile because it enables the use of tubes having different diameters.

The present invention has been described for illustrative but not limitative purposes, according to its preferred embodiments, but it is clear that modifications and/or changes can be introduced by those skilled in the art without departing from the relevant scope, as defined in the enclosed claims.

## Claims

1. Blade (200) of a laryngoscope, or similar devices, having a progressive spreading mechanism, said blade (200) **characterized in that** comprising:
- a lower part (201), preferably rectangular, which extends along the longitudinal direction and having a profile that is curved at the end (204), in turn comprising: a first dividing wall (208) positioned transversely to said lower part (201), which extends longitudinally up to a specific distance, and having a series of primary protruding teeth (212a, 212b, 212c, etc.) positioned along one or more columns, and that regulate the transverse thickness;
- an upper part (202), preferably rectangular, which extends along the longitudinal direction and having a profile that is curved at the end (203), in turn comprising: a second dividing wall (209) positioned transversely to said upper part (202), which extends longitudinally up to a specific distance, and having a series of secondary protruding teeth (218a, 218b, 218c, etc.) positioned along one or more columns, that regulate the transverse thickness, and interlocking to said primary protruding teeth (212a, 212b, 212c, etc.);
- a series of cylindrical elements (205a, 205b, 205c, etc.) that are all connected each other in correspondence of their axis, using a flexible articulated structure (206) free to move, under control of an operator, along the longitudinal direction, i.e. the main axis of the blade (200), inside an operative channel defined by a closed space between the above-mentioned lower part (201) and the upper part (202) of the blade (200),
so that the sliding and rolling motion of said cylindrical elements (205a, 205b, 205c, etc.), inside the above-mentioned operative channel, cause the spreading by contact of the space between said end (204) of the lower part (201) in respect to said end (203) of the upper part (202) of the blade (200), with a consequent increase of thickness of the distal part of the same blade (200).

2. Blade (200) of a laryngoscope, or similar devices, with a progressive spreading mechanism, according to previous claim, **characterized in that:**
- said lower part (201), said upper part (202) as well as the blade (200), the first dividing wall (208) and the second dividing wall (209), are composed of preformed metallic or plastic material that is easily adaptable to the specific anatomy of the patient.

3. Blade (200) of a laryngoscope, or similar devices, with a progressive spreading mechanism, according to one or more of previous claims, **characterized in that:**
- said series of cylindrical elements (205a, 205b, 205c, etc.) is composed entirely of cylindrical elements made of plastic, metallic or magnetic material, the latter being primarily used when said blade (200) is integrated within a magnetic guidance system.

4. Blade (200) of a laryngoscope, or similar devices, with a progressive spreading mechanism, according to one or more of previous claims, **characterized in that:**
- said cylindrical elements (205a, 205b, 205c, etc.) are arranged transversely to the longitudinal direction of the above-mentioned operative channel in which they are inserted, so that they can roll / slide inside it, and in such a way to allow a reduced friction during their motion.

5. Blade (200) of a laryngoscope, or similar devices, with a progressive spreading mechanism, according to one or more of previous claims, **characterized in that:**
- said structure (206) supporting the cylindrical elements (205a, 205b, 205c, etc.) is constituted by a series of elements that connect all the respective cylindrical elements (205a, 205b, 205c, etc.) that are rigid in the longitudinal direction, and articulated in the transverse direction; the same structure (206) ends, at the part that is closest to operator, with an upper surface that is coupled to gears of a toothed wheel; when the operator turns the toothed wheel backwards, the interconnected cylindrical elements (205a, 205b, 205c, etc.) advance deeply in the blade (200), when the operator turns the toothed wheel forwards the interconnected cylindrical elements (205a, 205b, 205c, etc.) move back towards the part that is closest to operator.

6. Blade (200) of a laryngoscope, or similar devices, with a progressive spreading mechanism, according to one or more of previous claims, **characterized in that:**
- the first dividing wall (208) presents, on the lateral surface, a series of primary protruding teeth (212a, 212b, 212c, etc.), and the second dividing wall (209) presents, on the lateral surface, a series of secondary protruding teeth (218a, 218b, 218c, etc.) positioned along one or more columns, that regulate the transverse thickness, and interlocking to said primary protruding teeth (212a, 212b, 212c, etc.), so that to adjust the spreading between said lower part (201) and said upper part (202) on different levels of size, depending on the diameter of the endotracheal tube (108) to be applied.

7. Blade (200) of a laryngoscope, or similar devices, with a progressive spreading mechanism, according to one or more of previous claims, **characterized in that:**
- said primary protruding teeth (212a, 212b, 212c, etc.) and said secondary protruding teeth (218a, 218b, 218c, etc.) are composed of the same metallic or plastic material that said lower part (201) and said upper part (202) are made, and their operation is based on a coupling mechanism that adjusts the spreading between the two parts (201) and (202) on different levels of size, depending on the diameter of the endotracheal tube (108) to be applied.

8. Blade (200) of a laryngoscope, or similar devices, with a progressive spreading mechanism, according to one or more of previous claims, **characterized in that:**
- said operative channel, in which the interconnected cylindrical elements (205a, 205b, 205c, etc.) are free to move, is perfectly / preferably parallel to a visual optical channel (210), suitable to transmit the images detected at the distal end of the blade (200).

9. Blade (200) of a laryngoscope, or similar devices, with a progressive spreading mechanism, according to one or more of previous claims, **characterized in that:**
- said operative channel, in which the interconnected cylindrical elements cylindrical elements (205a, 205b, 205c, etc.) are free to move, is located at the right side, from the point of view of the operator, in respect to said visual optical channel (210), or at the left side, in example in order to facilitate the left-handed operators.

10. Blade (200) of a laryngoscope, or similar devices, with a progressive spreading mechanism, according to one or more of previous claims, **characterized in that**:
- said operative channel and said visual optical channel (210) are aligned side by side, with said endotracheal tube (108) that is housed in the middle.

11. Blade (200) of a laryngoscope, or similar devices, with a progressive spreading mechanism, according to one or more of previous claims, **characterized in that:**
- said flexible articulated structure (206) is entirely extractable / removable from said operative channel so that when the endotracheal tube (108) has been properly positioned in the tracheal cavity the removal of the blade (200) from the patient's mouth is easier, safe and simple.

12. Blade (200) of a laryngoscope, or similar devices, with a progressive spreading mechanism, according to one or more of previous claims, **characterized in that:**
- said toothed wheel further includes with a locking / safety mechanism which makes it impossible, when activated, the retraction and/or the advancement of said interconnected cylindrical elements (205a, 205b, 205c, etc.).

13. Blade (200) of a laryngoscope, or similar devices, with a progressive spreading mechanism, according to one or more of previous claims, **characterized in that:**
- a disposable covering, preferably of plastic sterilized material, covers said blade (200), so that to prevent possible infections and / or contamination; the same blade (200) can be disposable.

14. Blade (200) of a laryngoscope, or similar devices, with a progressive spreading mechanism, according to one or more of previous claims, **characterized in that** further comprising:
- one or more protruding elements (210, 211) installed inside said operative channel so that, when they are intercepted by passage of said cylindrical elements (205a, 205b, 205c, etc.) they achieve an additional spreading of the upper part (203) and the lower part (204) at the distal end of blade (200), embedding a mechanism of profile variation similar to that of a McCoy *laryngoscope.*

15. Blade (200) of a laryngoscope, or similar devices, with a progressive spreading mechanism, according to one or more of previous claims, **characterized in that** further comprising:
- one or more protruding elements - that could be parallel each other, jointed to the upper part and/or to the lower part of blade, but coupled each other so that they take as less space as possible when said cylinders are at backward position in the operative channel - that achieve a further spreading of the distal end of blade (200) that is close to the *pharynx,* without an additional increase of size of the other end of blade (200) that is instead close to the *oral cavity.*
